# FASCICULE DE BREVET EUROPEEN

(11) **EP 4 178 409 B1**
(45) Date de publication et mention de la délivrance du brevet: **26.11.2025**
(21) Numéro de dépôt: 21742464.7
(22) Date de dépôt: 07.07.2021
(51) Int. Cl.: A61B 1/00

(54) **PISTON ENDOSCOPIQUE MEDICAL**
VENTIL FÜR EIN MEDIZINISCHES ENDOSKOP
MEDICAL ENDOSCOPE VALVE

(30) Priorité: 10.07.2020 FR 2007350
(43) Date de publication de la demande: 17.05.2023
(73) Titulaire: Advance Medical Integration Systems, 22950 Trégueux (FR)
(72) Inventeur: COLLIN, Hervé, 22800 QUINTIN (FR)
(74) Mandataire: IP Trust
(86) Numéro de dépôt international: PCT/FR2021/051259
(87) Numéro de publication internationale: WO 2022/008844

(56) Documents cités:
- WO-A1-2018/136274
- WO-A1-2019/072988
- WO-A1-2019/226307
- US-A1- 2015 144 215
- US-A1- 2015 216 393
- US-A1- 2017 143 194

## Description

### DOMAINE DE L'INVENTION

La présente invention concerne des pistons pour endoscopes, instruments médicaux qui servent à explorer les cavités d'organes internes.

Plus précisément, l'invention concerne les pistons, généralement à usage unique, commandant le débit d'air ou d'eau. Les pistons d'aspiration et d'insufflation/lavage sont des pièces comprenant plusieurs pièces de nature différentes, notamment un piston cylindrique, un corps pour le montage sur la poignée d'un endoscope et un ressort interposé entre le corps et le piston pour assurer la fonction d'obturation ou d'ouverture des lumières prévues dans le corps de la poignée, ainsi que la force de rappel permettant à l'utilisateur d'exercer la pression de manière ergonomique et maîtrisée.

### ETAT DE LA TECHNIQUE

On connaît dans l'état de la technique le brevet américain US2017/143194 concernant une vanne d'air/eau jetable pour un endoscope, comprenant une tige comportant un passage d'une ouverture vers un évent. La tige comporte des rainures et des arêtes et une protubérance formée au niveau de l'évent. Des joints d'étanchéité sont disposés dans certaines des rainures. Un anneau interne, comportant un diaphragme, s'étend d'une circonférence externe de l'anneau interne à une circonférence interne d'un capuchon externe. Des charnières s'étendent verticalement vers le bas depuis le diaphragme et des nervures sont formées le long de la circonférence interne du capuchon externe. Un capuchon de bouton comporte un anneau interne qui se fixe rigidement à la tige. Un élément élastique est disposé de façon fixe entre le capuchon de bouton et le diaphragme. Le capuchon externe, l'anneau interne et l'anneau interne du capuchon de bouton définissent un alésage central pour recevoir la tige dans celui-ci.

La demande de brevet PCT WO2019/226307 décrit un adaptateur de nettoyage pour un endoscope. L'adaptateur de nettoyage comprend une tige principale comprenant un premier trou traversant s'étendant transversalement à travers la tige principale, un second trou traversant s'étendant transversalement à travers la tige principale, et un canal à l'intérieur de la tige principale couplant de manière fluidique le premier trou traversant au second trou traversant. Dans certains modes de réalisation, l'invention concerne une étiquette pour une valve d'endoscope. L'invention concerne également des procédés et des kits.

La demande de brevet PCT WO2018/136274 décrit un ensemble valve jetable conçu pour être utilisé avec un endoscope. L'ensemble valve jetable peut comprendre une tige composée d'un matériau thermoplastique, un étançon à ressort configuré pour recevoir la tige et permettre le mouvement de la tige dans une position ascendante et descendante par rapport à l'étançon à ressort, un ressort configuré pour mettre en contact ledit étançon à ressort avec la tige et un lubrifiant disposé sur la tige, l'étançon à ressort et/ou le ressort. L'ensemble valve jetable peut également comprendre un agent antimicrobien disposé dans le lubrifiant, ou dans le matériau thermoplastique et/ou pouvant être revêtu dessus. Un procédé de fabrication d'ensembles valves jetables destinés à être utilisés avec un endoscope peut comprendre plusieurs étapes.

### Inconvénients de l'art antérieur

Les solutions de l'art antérieur sont constituées par un assemblage de pièces métalliques (le ressort) et en plastique (le corps, la tige et l'embase d'appui). L'assemblage de telles pièces nécessite des interventions essentiellement manuelles peut compatibles avec la préparation de pièces stériles. Elles présentent par ailleurs des zones de raccordement difficile à stériliser. La stérilisation est généralement réalisée à l'aide d'un stérilisateur à EtO (oxyde d'éthylène) pendant une durée suffisante pour que le gaz puisse pénétrer dans tous les interstices.

### Solution apportée par l'invention

La présente invention vise à éviter les inconvénients de l'art antérieur en proposant un piston pour endoscope, de préférence à usage unique et jetable, facile à réaliser, d'un coût de fabrication réduit et conférant à l'utilisateur un maniement ergonomique, stérilisable à l'EtO.

L'invention concerne selon son acception la plus générale un piston pour assurer la fonction d'obturation ou d'ouverture des lumières prévues dans le corps de la poignée d'un endoscope, formé par :
- une tige présentant un canal central débouchant dans la surface frontale proximale d'une part, et dans un conduit transversal d'autre part,
- un poussoir moulé en une seule pièce, formée par deux jupes annulaires coaxiales, la jupe extérieure présentant une embase annulaire configurée pour l'engagement sur ledit corps de la poignée, lesdites jupes étant reliées par au moins une liaison déformable élastiquement pour permettre un déplacement axial relatif entre-elles
- la jupe intérieure dudit poussoir étant enchâssée sur le tronçon proximal de ladite tige.

De préférence, ladite tige présente des joints surmoulés sur des gorges annulaires. De préférence, lesdits joints présentent une forme lenticulaire et sont constitué en un matériau de surmoulage moins dur que le matériau de la tige.

Selon un mode de réalisation préféré, la liaison entre lesdites jupes est assurée par deux liaisons diamétralement opposées. Avantageusement, ladite liaison déformable élastiquement est constituée par une patte recourbée dont l'un des bords transversaux est solidaire de ladite jupe extérieure et l'autre bord transversal est solidaire de ladite jupe intérieure. Selon une variante ladite jupe extérieure présente une lumière périphérique pour permettre le débattement latéral de ladite patte lors du déplacement axial relatif desdites jupes.

Selon une variante, ladite jupe intérieure présente également une fenêtre périphérique, et en ce que ladite patte relie le bord transversal de la lumière de la jupe extérieure (60) au bord transversal de la lumière de la jupe intérieure.

De préférence, ledit piston est configuré pour coopérer avec le corps de vanne d'aspiration d'une poignée d'un endoscope. Avantageusement ledit piston est configuré pour se coopérer avec le corps de vanne air/eau d'une poignée d'un endoscope. L'invention concerne aussi un ensemble à usage unique formé par un piston d'aspiration et un piston air/eau pour endoscope caractérisé en ce que chacun des pistons est constitué par :
- une tige présentant un canal central débouchant dans la surface frontale proximale d'une part, et dans un conduit transversal d'autre part,
- un poussoir moulé en une seule pièce, formée par deux jupes annulaires coaxiales, la jupe extérieure présentant une embase annulaire configurée pour l'engagement sur ledit corps de la poignée, lesdites jupes étant reliées par au moins une liaison déformable élastiquement pour permettre un déplacement axial relatif entre-elles
- la jupe intérieure dudit poussoir étant enchâssée sur le tronçon proximal de ladite tige.

L'invention concerne aussi un procédé de fabrication d'un piston à usage unique pour endoscope consistant
- à injecter dans une première empreinte de moulage une première matière plastique élastomère dans un moule pour le moulage d'une tige présentant un canal central débouchant dans la surface frontale proximale d'une part, et dans un conduit transversal d'autre part, à injecter une seconde matière plastique élastomère dans un moule pour le surmoulage de joints
- et à injecter dans deuxième empreinte de moulage une matière plastique élastomère pour former ladite jupe intérieure dudit poussoir
- et à enchâsser ledit poussoir sur le tronçon proximal de ladite tige.

Avantageusement, le moulage de ladite tige et le moulage dudit poussoir sont réalisés pendant un même cycle d'injection, et en ce que ledit poussoir est enchâssé sur ledit proximal de ladite tige avant le refroidissement des pièces moulées.

### Description détaillée d'un exemple non limitatif de réalisation

L'invention est décrite ci-après, à titre d'exemple non limitatif, par référence aux dessins annexés dans lesquels :
[Fig. 1] la figure 1 est une vue en perspective partielle éclatée montrant une poignée d'endoscope avec le couple de piston objet de l'invention
[Fig. 2] la figure 2 est une vue en perspective d'un piston d'aspiration selon l'invention
[Fig. 3] la figure 3 représente vue éclatée d'un piston d'aspiration selon l'invention
[Fig. 4] la figure 4 représente vue en coupe d'un piston d'aspiration selon l'invention
[Fig. 5] la figure 5 représente vue en perspective du poussoir d'un piston d'aspiration selon l'invention
[Fig. 6] la figure 6 représente vue en coupe du poussoir d'un piston d'aspiration selon l'invention

### Contexte de l'invention

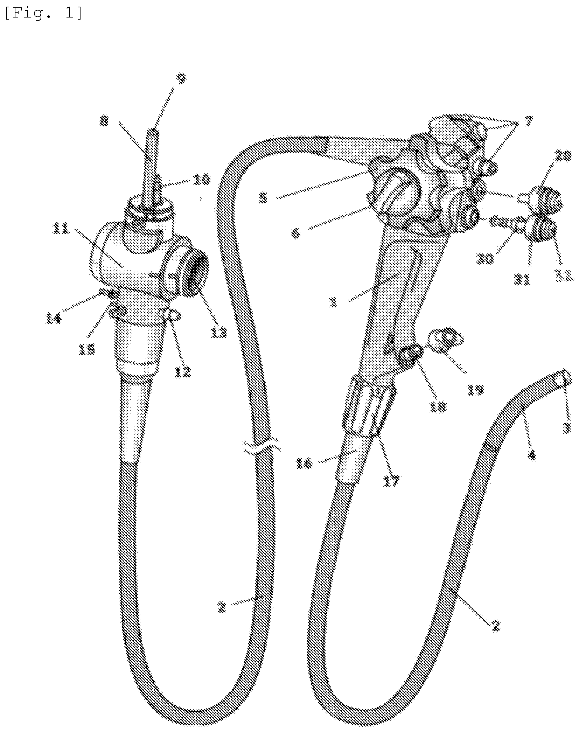

La figure 1 représente un exemple d'endoscope connu. Il comporte une poignée (1) à partir de laquelle s'étend un tube souple (2) se terminant par une extrémité distale ou opérationnelle (3) qui est introduite dans la cavité à explorer et dont l'orientation peut être commandée par un manchon béquillable (4). Ce manchon est commandé par des fils qui sont tirés de manière contrôlée par suite de l'actionnement manuel de molettes ou de boutons (5, 6, 7) prévus sur la poignée (1) d'endoscope. La section de commande des manettes mobiles permet au médecin de commander toutes les fonctions de l'endoscope. Les manettes (5, 6, 7) de béquillage (orientation dans l'espace) dirigent les câbles de béquillage et contrôlent la section béquillable à l'extrémité distale du tube d'insertion, permettant ainsi une orientation bidimensionnelle. Des mécanismes de blocage (freins) permettent de fixer la section béquillable dans la position voulue.

La lumière est transmise par une fibre optique (8) dont l'extrémité proximale (9) est accouplée à un connecteur optique. Le bloc de raccordement (11) comprend en outre une prise d'air (10).

Un raccord pneumatique (12) est destiné à être raccordé à une pompe à vide ou une pompe à air réversible pour commander l'insufflation ou l'aspiration à l'extrémité distale (3). Le bloc technique (11) comporte également un connecteur électrique (13) et un support de raccordement (14) d'un cordon de sécurité et une alimentation en eau (15).

La poignée (1) est raccordé sur le tube (2) par une gaine (16) prolongée par un manchon (17). La poignée comporte une ouverture du canal opérateur (18) équipée d'une valve piston à biopsie jetable (19).

La poignée comporte un piston (20) commandant le canal de lavage et un piston (30) de commande de l'aspiration ou d'insufflation par l'intermédiaire de deux tubes s'étendant entre la poignée (1) et l'extrémité distale (3), l'un pour amener de l'air et de l'eau à ladite extrémité distale, l'autre pour effectuer une biopsie ou une aspiration. L'application d'une aspiration est réglée au moyen d'un piston (30) prévue sur la poignée. Le piston est réuni à un conduit voisin par des liaisons soudées. Les pistons (20, 30) sont accouplés sur le corps de la poignée par des embases annulaires (32).

Le piston (30) relie le canal d'aspiration au canal opérateur dans le tube d'insertion. En appuyant sur le bouton (31) du piston, on peut effectuer une aspiration du canal opérateur. Le piston air/eau (20) est semblable au piston et au piston d'aspiration (30), sauf qu'un piston avec bouton à deux voies est utilisée dans un dispositif à double canal qui permet de véhiculer l'air ou l'eau jusqu'à la lentille à l'extrémité distale, pour le lavage ou l'insufflation d'air afin d'améliorer la vision. Les deux pistons (20, 30) peuvent être détachés pour le remplacement, lorsqu'ils sont à usage unique, ou sinon pour le nettoyage.

### Description détaillée des pistons (20, 30)

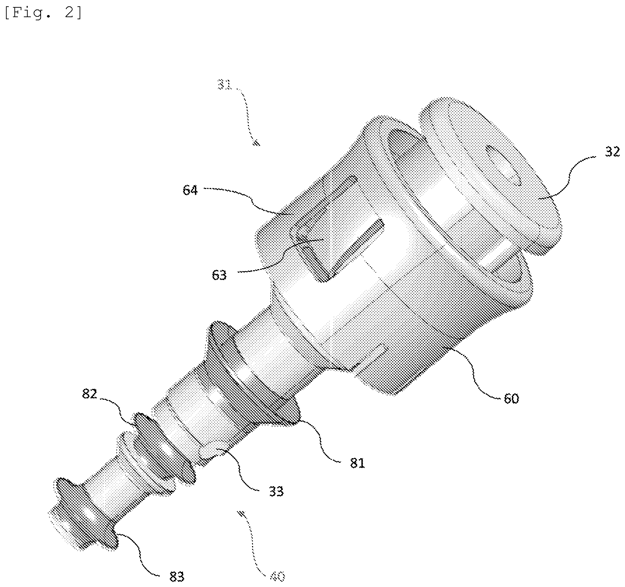

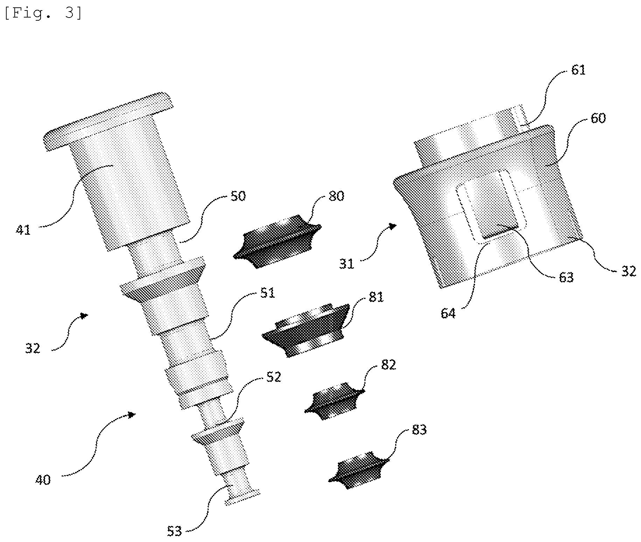

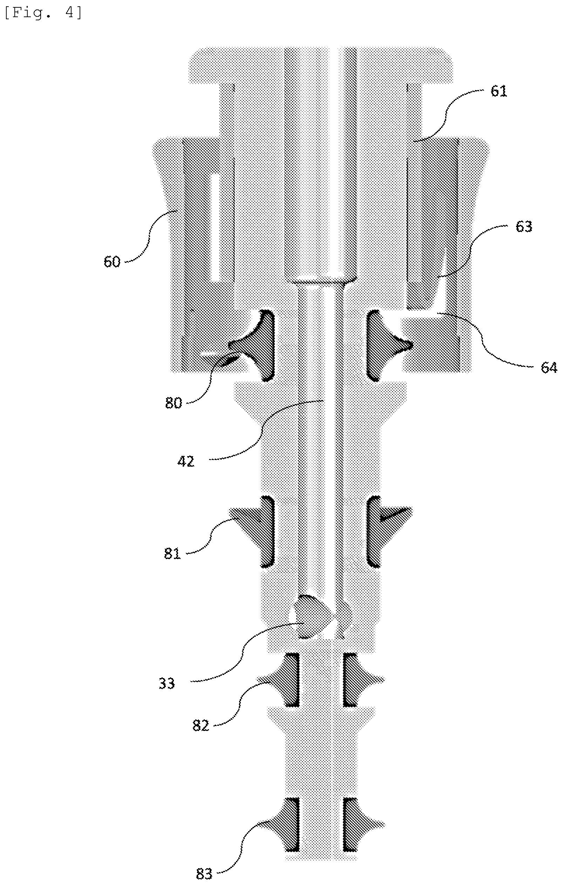

Les figures 2 à 4 représentent différentes vues d'un piston d'aspiration conforme à l'invention. Selon un aspect de l'invention, la tige (40) de piston pour endoscope est en matière plastique synthétique moulée et présente un conduit rectiligne (42) débouchant dans un conduit transversal (33). Cette pièce présente une forme cylindrique avec plusieurs gorges annulaires (50 à 53) dans lesquelles on injecte ensuite une deuxième matière plastique pour la réalisation par surmoulage des joints (80 à 83). L'ensemble forme ainsi une pièce monolithique surmoulée, présentant les fonctions nécessaires pour la fermeture étanche et l'ouverture d'un conduit prévu dans l'endoscope. La tige (40) de piston est communément moulée en polypropylène.

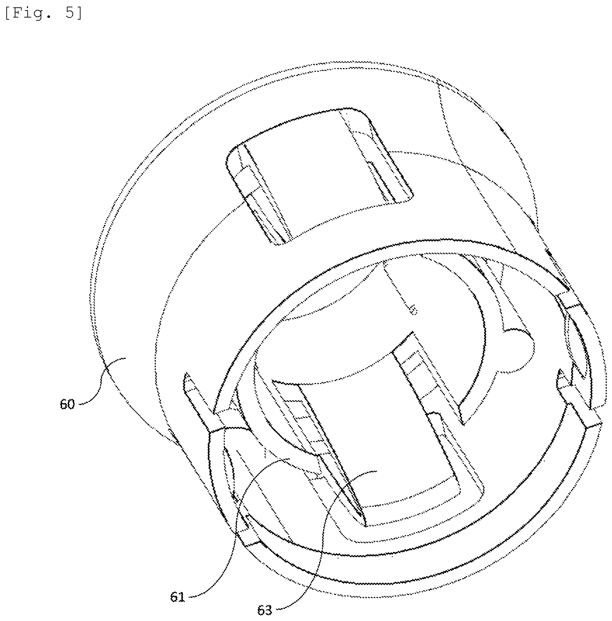

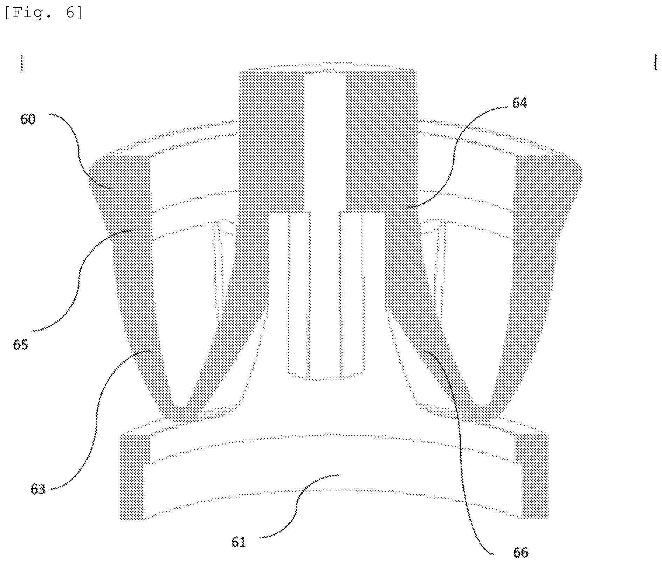

Le poussoir (31) constitue une deuxième pièce monolithique également réalisée par moulage. Comme cela est visible sur les figures 5 à 6, il présente une jupe extérieure (60) annulaire et une jupe intérieure (61) annulaire, coaxiales.

Les deux jupes (60, 61) sont reliées par deux pattes (63) déformables élastiquement pour permettre un déplacement relatif selon une direction axiale des deux jupes (60, 61).

Ces pattes (63) sont recourbées et présente une forme en « U », un premier bord transversal (65) étant relié à la jupe extérieure (60) et le second bord transversal (64) étant relié à la jupe intérieure (61). Les jupes présentent des fenêtres permettant le débattement des pattes lorsqu'un effort est exercé sur le poussoir (31). Ces pattes (63, 66) forment des ressorts assurant le guidage élastique du poussoir (31).

### Procédé de fabrication

La fabrication du piston peut être réalisée par moulage dans un moule à tiroirs, pour le moulage de la tige (40) puis la formation des joints (80 à 83) par surmoulage avec une seconde injection d'une matière mois dure que celle utilisée pour l'injection du noyau de la tige.

Le poussoir est réalisé pendant le même cycle d'injection dans un autre moule. Il est ensuite déplacé par un bras robotisé pour être engagé sur la tige (40) et enchâssée sur le tronçon proximal avant refroidissement des pièces.

Les pistons selon l'invention peuvent être réunis dans une poche stérile, pour former un kit de pistons à usage unique.
être engagé sur la tige (40) et enchâssée sur le tronçon proximal avant refroidissement des pièces.

Les pistons selon l'invention peuvent être réunis dans une poche stérile, pour former un kit de pistons à usage unique.

## Revendications

1. - Piston (20, 30) pour assurer la fonction d'obturation ou d'ouverture des lumières prévues dans le corps de la poignée d'un endoscope, comprenant une tige (40) présentant une forme cylindrique avec plusieurs gorges annulaires (50 à 53) de piston pour endoscope est en matière plastique synthétique moulée et présentant un conduit rectiligne (42) débouchant dans un conduit transversal (33), et un poussoir (31) ledit piston étant formé par l'assemblage de deux pièces en matière plastique moulées monolithiques constituées par :
- Une première pièce monolithique constituée par ladite tige (40), adaptée pour la fermeture étanche et l'ouverture d'un conduit prévu dans l'endoscope, ladite tige (40) présentant un canal central débouchant dans une surface frontale proximale (32) d'une part, et dans le conduit transversal (33) d'autre part,
- une deuxième pièce monolithique constituée par ledit poussoir (31) présentant une jupe extérieure (60) annulaire et une jupe intérieure (61) annulaire, coaxiales, reliées par deux pattes (63) déformables élastiquement pour permettre un déplacement relatif selon une direction axiale des deux jupes (60, 61), lesdites pattes (63) étant recourbées et présentant une forme en « U », un premier bord transversal (65) étant relié à la jupe extérieure (60) et le second bord transversal (64) étant relié à la jupe intérieure (61), lesdites jupes (60, 61) présentant des fenêtres permettant le débattement des pattes lorsqu'un effort est exercé sur le poussoir (31), lesdites pattes (63, 66) formant des ressorts assurant le guidage élastique du poussoir (31)
- la jupe intérieure (61) dudit poussoir (31) étant enchâssée sur le tronçon proximal (41) de ladite tige (40).

2. - Piston pour assurer la fonction d'obturation ou d'ouverture des lumières prévues dans le corps de la poignée médical d'un endoscope, selon la revendication 1 **caractérisé en ce que** ladite tige (40) présente des joints (80 à 83) surmoulés sur des gorges annulaires (50 à 53).

3. - Piston pour assurer la fonction d'obturation ou d'ouverture des lumières prévues dans le corps de la poignée médical d'un endoscope, selon la revendication précédente **caractérisé en ce que** lesdits joints (80 à 83) présentent une forme lenticulaire et sont constitué en un matériau de surmoulage moins dur que le matériau de la tige (40).

4. - Piston pour assurer la fonction d'obturation ou d'ouverture des lumières prévues dans le corps de la poignée médical d'un endoscope, selon la revendication 1 **caractérisé en ce que** la liaison entre lesdites jupes (60, 61) est assurée par deux liaisons (63) diamétralement opposées.

5. - Piston pour assurer la fonction d'obturation ou d'ouverture des lumières prévues dans le corps de la poignée médical d'un endoscope, selon la revendication 1 **caractérisé en ce que** ladite liaison déformable élastiquement est (63) constituée par une patte recourbée dont l'un des bords transversaux est solidaire de ladite jupe extérieure (60) et l'autre bord transversal est solidaire de ladite jupe intérieure (61).

6. - Piston pour assurer la fonction d'obturation ou d'ouverture des lumières prévues dans le corps de la poignée médical d'un endoscope, selon la revendication précédente **caractérisé en ce que** ladite jupe extérieure (60) présente une lumière (64) périphérique pour permettre le débattement latéral de ladite patte (63) lors du déplacement axial relatif desdites jupes (60, 61).

7. - Piston pour assurer la fonction d'obturation ou d'ouverture des lumières prévues dans le corps de la poignée médical d'un endoscope, selon la revendication précédente **caractérisé en ce que** ladite jupe intérieure (61) présente également une fenêtre périphérique, et **en ce que** ladite patte (63) relie le bord transversal de la lumière (63) de la jupe extérieure (60) au bord transversal de la lumière (63) de la jupe intérieure (61).

8. - Piston pour assurer la fonction d'obturation ou d'ouverture des lumières prévues dans le corps de la poignée médical d'un endoscope, selon la revendication 1 **caractérisé en ce qu'**il est configuré pour coopérer avec le corps de vanne d'aspiration d'une poignée d'un endoscope.

9. - Piston pour assurer la fonction d'obturation ou d'ouverture des lumières prévues dans le corps de la poignée médical d'un endoscope, selon la revendication 1 **caractérisé en ce qu'**il est configuré pour se coopérer avec le corps de vanne air/eau d'une poignée d'un endoscope.

10. - Ensemble à usage unique formé par un piston d'aspiration et un piston air/eau pour endoscope, chacun des pistons conforme à la revendication 1 étant constitué par :
- une tige (40) présentant un canal central débouchant dans la surface frontale proximale (32) d'une part, et dans un conduit transversal (33) d'autre part,
- un poussoir (31) moulé en une seule pièce, formée par deux jupes annulaires coaxiales (60, 61), la jupe extérieure (60) présentant une embase annulaire (32) configurée pour l'engagement sur ledit corps de la poignée, lesdites jupes (60, 61) étant reliées par au moins une liaison déformable élastiquement (63) pour permettre un déplacement axial relatif entre-elles
- la jupe intérieure (61) dudit poussoir (60) étant enchâssée sur le tronçon proximal (41) de ladite tige (40).

11. - Procédé de fabrication d'un piston à usage unique pour endoscope conforme à la revendication 1, consistant
- à injecter dans une première empreinte de moulage une première matière plastique élastomère dans un moule pour le moulage d'une tige (40) présentant un canal central débouchant dans la surface frontale proximale (32) d'une part, et dans un conduit transversal (33) d'autre part, à injecter une seconde matière plastique élastomère dans un moule pour le surmoulage de joints (80 à 83)
- et à injecter dans deuxième empreinte de moulage une matière plastique élastomère pour former ladite jupe intérieure (61) dudit poussoir (31)
- et à enchâsser ledit poussoir (31) sur le tronçon proximal (41) de ladite tige (40).

12. - Procédé de fabrication d'un piston à usage unique selon la revendication précédente **caractérisé en ce que** le moulage de ladite tige (40) et le moulage dudit poussoir (31) sont réalisés pendant un même cycle d'injection, et **en ce que** ledit poussoir (31) est enchâssé sur ledit proximal (41) de ladite tige (40) avant le refroidissement des pièces moulées.

## Patentansprüche

1. Kolben (20, 30) zum Verschließen oder Öffnen der im Griffkörper eines Endoskops vorgesehenen Lumen, umfassend einen Schaft (40), der eine zylindrische Form mit mehreren Ringnuten (50 bis 53) aufweist, wobei der Kolben für ein Endoskop aus synthetischem Kunststoff geformt ist und einen geradlinigen Kanal (42) aufweist, der in einen Querkanal (33) mündet, und einem Drücker (31),
wobei der Kolben durch das Zusammenfügen von zwei monolithisch geformten Kunststoffteilen gebildet wird, gebildet aus:
- Einem ersten monolithischen Teil, das aus dem genannten Schaft (40) gebildet ist und zum dichten Verschließen und Öffnen eines im Endoskop vorgesehenen Kanals geeignet ist, wobei der Schaft (40) einen zentralen Kanal aufweist, der einerseits in eine proximale Stirnfläche (32) und andererseits in den Querkanal (33) mündet,
- einem zweiten monolithischen Teil, das durch den genannten Drücker (31) gebildet ist, der eine äußere ringförmige Schürze (60) und eine innere ringförmige Schürze (61) aufweist, die koaxial sind und durch zwei elastisch verformbare Laschen (63) verbunden sind, um eine relative Verschiebung gemäß einer axialen Richtung der beiden Schürzen (60, 61) zu ermöglichen,
wobei die Laschen (63) gebogen sind und eine "U"-Form aufweisen, wobei eine erste Querkante (65) mit der Außenschürze (60) verbunden ist und die zweite Querkante (64) mit der Innenschürze (61) verbunden ist,
wobei die Schürzen (60, 61) Fenster aufweisen, die das Ausschwenken der Laschen ermöglichen, wenn eine Kraft auf den Drücker (31) ausgeübt wird, wobei die Laschen (63, 66) Federn bilden, die die elastische Führung des Drückers (31) gewährleisten,
- wobei die innere Schürze (61) des Drückers (31) auf dem proximalen Abschnitt (41) des Schafts (40) eingebettet ist.

2. Kolben, um die Funktion des Verschließens oder Öffnens der im Körper des medizinischen Griffs eines Endoskops vorgesehenen Lumen zu gewährleisten, nach Anspruch 1, **dadurch gekennzeichnet, dass** der Schaft (40) Dichtungen (80 bis 83) aufweist, die auf ringförmige Nuten (50 bis 53) aufgeformt sind.

3. Kolben, um die Funktion des Verschließens oder Öffnens der im Körper des medizinischen Griffs eines Endoskops vorgesehenen Lumen zu gewährleisten, nach dem vorhergehenden Anspruch, **dadurch gekennzeichnet, dass** die Dichtungen (80 bis 83) eine linsenförmige Form aufweisen und aus einem Überformungsmaterial gebildet sind, das weniger hart ist als das Material des Schafts (40).

4. Kolben, um die Funktion des Verschließens oder Öffnens der im Körper des medizinischen Griffs eines Endoskops vorgesehenen Lumen zu gewährleisten, nach Anspruch 1, **dadurch gekennzeichnet, dass** die Verbindung zwischen den Schürzen (60, 61) durch zwei diametral gegenüberliegende Verbindungen (63) gewährleistet wird.

5. Kolben, um die Funktion des Verschlusses oder der Öffnung der im Körper des medizinischen Griffs eines Endoskops vorgesehenen Lumen zu gewährleisten, nach Anspruch 1, **dadurch gekennzeichnet, dass** die elastisch verformbare Verbindung (63) aus einer gebogenen Lasche gebildet ist, von der eine der Querkanten fest mit der äußeren Schürze (60) und die andere Querkante fest mit der inneren Schürze (61) verbunden ist.

6. Kolben, um die Funktion des Verschließens oder Öffnens der im Körper des medizinischen Griffs eines Endoskops vorgesehenen Lumen zu gewährleisten, nach dem vorhergehenden Anspruch, **dadurch gekennzeichnet, dass** die äußere Schürze (60) ein peripheres Lumen (64) aufweist, um die seitliche Auslenkung der Lasche (63) bei der axialen Relativbewegung der Schürzen (60, 61) zu ermöglichen.

7. Kolben, um die Funktion des Verschließens oder Öffnens der im Körper des medizinischen Griffs eines Endoskops vorgesehenen Lumen zu gewährleisten, nach dem vorhergehenden Anspruch, **dadurch gekennzeichnet, dass** die innere Schürze (61) ebenfalls ein Umfangsfenster aufweist, und dass die Lasche (63) die Querkante des Lumens (63) der äußeren Schürze (60) mit der Querkante des Lumens (63) der inneren Schürze (61) verbindet.

8. Kolben, um die Funktion des Verschließens oder Öffnens der im Körper des medizinischen Griffs eines Endoskops vorgesehenen Lumen zu gewährleisten, nach Anspruch 1, **dadurch gekennzeichnet, dass** er konfiguriert ist, um mit dem Körper des Saugventils eines Griffs eines Endoskops zusammenzuwirken.

9. Kolben, um die Funktion des Verschließens oder Öffnens der Lumen zu gewährleisten, die im Körper des medizinischen Griffs eines Endoskops vorgesehen sind, nach Anspruch 1, **dadurch gekennzeichnet, dass** er so konfiguriert ist, dass er mit dem Luft/Wasser-Ventilkörper eines Griffs eines Endoskops zusammenwirkt.

10. Einweg-Set gebildet aus einem Saugkolben und einem Luft-/Wasserkolben für Endoskope, wobei jeder der Kolben gemäß Anspruch 1 gebildet ist aus:
- einen Schaft (40) mit einem zentralen Kanal, der einerseits in die proximale Vorderfläche (32) und andererseits in einen Querkanal (33) mündet,
- einen einstückig gegossenen Drücker (31), der aus zwei koaxialen ringförmigen Schürzen (60, 61) gebildet ist, wobei die äußere Schürze (60) einen ringförmigen Ansatz (32) aufweist, der für den Eingriff mit dem Körper des Griffs konfiguriert ist, wobei die Schürzen (60, 61) durch mindestens eine elastisch verformbare Verbindung (63) verbunden sind, um eine relative axiale Verschiebung zwischen ihnen zu ermöglichen
- wobei die innere Schürze (61) des Drückers (60) auf dem proximalen Abschnitt (41) des Schafts (40) eingebettet ist.

11. Verfahren zur Herstellung eines Einwegkolbens für ein Endoskop gemäß Anspruch 1, bestehend
- aus Einspritzen in einen ersten Formhohlraum ein erstes elastomeres Kunststoffmaterial in eine Form zum Gießen des Schafts (40), die einen zentralen Kanal aufweist, der einerseits in die proximale Stirnfläche (32) und andererseits in einen Querkanal (33) mündet, aus Einspritzen eines zweiten elastomeren Kunststoffmaterials in eine Form zum Übergießen von Dichtungen (80 bis 83)
- und aus Einspritzen eines elastomeren Kunststoffmaterials in den zweiten Formhohlraum, um die Innenschürze (61) des Drückers (31) zu bilden
- und den Drücker (31) auf den proximalen Abschnitt (41) des Schafts (40) aufzustecken.

12. Verfahren zur Herstellung eines Einwegkolbens nach dem vorhergehenden Anspruch, **dadurch gekennzeichnet, dass** das Formen des Schafts (40) und das Formen des Drückers (31) während desselben Spritzzyklus durchgeführt werden und dass der Drücker (31) vor dem Abkühlen der geformten Teile auf den Proximalteil (41) des Schafts (40) aufgezogen wird.

## Claims

1. Valve (20, 30) for providing the function of closing or opening the apertures provided in the body of the handle of an endoscope, comprising a rod (40) having a cylindrical shape with several annular grooves (50 to 53) of an endoscope valve is made of moulded synthetic material and having a straight duct (42) opening into a transverse duct (33), and a pusher (31),
said valve being formed by the assembly of two monolithic moulded plastic parts consisting of:
- A first monolithic part consisting of said rod (40), suitable for sealing and opening a duct provided in the endoscope, said rod (40) having a central channel opening into a proximal front surface (32) on the one hand, and into the transverse duct (33) on the other hand,
- a second monolithic part consisting of said pusher (31) having an annular outer skirt (60) and an annular inner skirt (61), coaxial, connected by two tabs (63) elastically deformable to allow a relative movement in an axial direction of the two skirts (60, 61), said tabs (63) being curved and having a "U" shape, a first transverse edge (65) being connected to the outer skirt (60) and the second transverse edge (64) being connected to the inner skirt (61),
said skirts (60, 61) having windows allowing the movement of the tabs when a force is exerted on the pusher (31), said tabs (63, 66) forming springs ensuring the elastic guidance of the pusher (31),
- the inner skirt (61) of said pusher (31) being embedded on the proximal (41) section of said rod (40).

2. Valve for providing the function of closing or opening the apertures provided in the body of the medical handle of an endoscope, according to claim 1, **characterised in that** said rod (40) has seals (80 to 83) overmoulded on annular grooves (50 to 53).

3. Valve for providing the function of closing or opening the apertures provided in the body of the medical handle of an endoscope, according to the preceding claim, **characterised in that** said seals (80 to 83) have a lenticular shape and consist of an overmoulding material less hard than the material of the rod (40).

4. Valves for providing the function of closing or opening the apertures provided in the body of the medical handle of an endoscope, according to claim 1, **characterised in that** the connection between said skirts (60, 61) is provided by two diametrically opposed connections (63).

5. Valve for providing the function of closing or opening the apertures provided in the body of the medical handle of an endoscope, according to claim 1, **characterised in that** said elastically deformable connection is (63) formed by a curved tab of which one of the transverse edges is secured to said outer skirt (60) and the other transverse edge is secured to said inner skirt (61).

6. Valve for providing the function of closing or opening the apertures provided in the body of the medical handle of an endoscope, according to the preceding claim, **characterised in that** said outer skirt (60) has a peripheral aperture (64) to allow lateral movement of said tab (63) during the relative axial movement of said skirts (60, 61).

7. Valve for providing the function of closing or opening the apertures provided in the body of the medical handle of an endoscope, according to the preceding claim, **characterised in that** said inner skirt (61) also has a peripheral window, and **in that** said tab (63) connects the transverse edge of the aperture (63) of the outer skirt (60) to the transverse edge of the aperture (63) of the inner skirt (61).

8. Valve for providing the function of closing or opening the apertures provided in the body of the medical handle of an endoscope, according to claim 1, **characterised in that** it is configured to cooperate with the suction valve body of a handle of an endoscope.

9. Valve for providing the function of closing or opening the apertures provided in the body of the medical handle of an endoscope, according to claim 1, **characterised in that** it is configured to cooperate with the air/water valve body of a handle of an endoscope.

10. Single-use assembly formed by a suction valve and an air/water valve for an endoscope, each of the valves according to claim 1 consisting of:
- a rod (40) having a central channel opening into the proximal front surface (32) on the one hand, and into a transverse duct (33) on the other hand,
- a pusher (31) moulded in one piece, formed by two coaxial annular skirts (60, 61), the outer skirt (60) having an annular base (32) configured for engagement on said body of the handle, said skirts (60, 61) being connected by at least one elastically deformable connection (63) to allow an axial movement relative to each other,
- the inner skirt (61) of said pusher (60) being embedded on the proximal (41) section of said rod (40).

11. Method for manufacturing a single-use endoscope valve according to claim 1, consisting
- of injecting into a first moulding cavity a first elastomeric plastic material in a mould for moulding a rod (40) having a central channel opening into the proximal front surface (32) on the one hand, and into a transverse duct (33) on the other hand, injecting a second elastomeric plastic material into a mould for overmoulding seals (80 to 83)
- and injecting into a second moulding cavity an elastomeric plastic material to form said inner skirt (61) of said pusher (31)
- and embedding said pusher (31) on the proximal (41) section of said rod (40).

12. Method for manufacturing a single-use valve according to the preceding claim, **characterised in that** the moulding of said rod (40) and the moulding of said pusher (31) are carried out during the same injection cycle, and **in that** said pusher (31) is embedded on said proximal (41) of said rod (40) before cooling the moulded parts.
